Europäisches Patentamt

European Patent Office

Office européen des brevets

(19)

(11) Publication number: **0 399 819 B1**

# EUROPEAN PATENT SPECIFICATION

(45) Date of publication of patent specification: **10.05.95**

(51) Int. Cl.⁶: **C12N 1/20**, C12N 3/00, A23K 1/00, A23K 1/16, //(C12N1/20,C12R1:07)

(21) Application number: **90305644.8**

(22) Date of filing: **23.05.90**

(54) **Stabilised spore-forming viable microorganisms preparation, its production and pellet thereof.**

(30) Priority: **24.05.89 JP 130580/89**
**06.03.90 JP 54711/90**

(43) Date of publication of application:
**28.11.90 Bulletin 90/48**

(45) Publication of the grant of the patent:
**10.05.95 Bulletin 95/19**

(84) Designated Contracting States:
**AT BE CH DE DK ES FR GB GR IT LI LU NL SE**

(56) References cited:
**EP-A- 0 042 303**
**EP-A- 0 257 996**
**EP-A- 0 281 091**
**EP-A- 0 296 051**
**CH-A- 339 326**

**PATENT ABSTRACTS OF JAPAN vol.12, no.283 (C-518), 3 August 1988; & JP-A-63063620 (TOA YAKUHIN KOGYO KK) 22.03.1988**

(73) Proprietor: **ASAHI KASEI KOGYO KABUSHIKI KAISHA**
**2-6, Dojimahama 1-chome**
**Kita-ku**
**Osaka (JP)**

(72) Inventor: **Suzuki,Masaki 607-3-2 Shimo-Funabara,**
**Yugashima-Cho**
**Tagata-gun**
**Shizuoka (JP)**
Inventor: **Yamaoka,Hideyuki 883 Mifuku,Ohito-cho,**
**Tagata-gun**
**Shizuoka (JP)**
Inventor: **Aoshima,Mutsumi 883,Mifuku Ohito-cho**
**Tagata-gun**
**Shizuoka (JP)**
Inventor: **Hashimoto,Koji 815-22, Nitta, Kan-nami-cho**
**Tagata-gun**
**Shizuoka (JP)**

EP 0 399 819 B1

PATENT ABSTRACTS OF JAPAN vol. 7, no. 268 (C-197), 30 November 1983; & JP - A - 58149675 (MEIJI NIYUUGIYOU KK) 06.09.1983

PATENT ABSTRACTS OF JAPAN vol. 9, no. 271 (C-311)(1994), 29 October 1985; & JP - A - 60122098 (KENJI ICHIKAWA) 29.06.1985

(74) Representative: **Woods, Geoffrey Corlett et al**
**J.A. KEMP & CO.**
**14 South Square**
**Gray's Inn**
**London WC1R 5LX (GB)**

**Description**

This invention relates to a stabilized spore-forming viable microorganism preparation, its production and pellets thereof.

A viable microorganism preparation for feed is a feed in which viable and useful microorganisms are mixed with feed, and is frequently used for prevention and treatment of animal diseases or improvement in animal health. An enteric bacterium, Bifidobacterium, viable in the intestine, is known to play an important role for prevention of intestinal infection, intestinal putrefaction and diarrhoea, and food and feed added with viable Bifidobacterium are commercially available. A bacterium belonging to the genus Bacillus is also known to be useful, and feed containing the said bacteria is known. In these viable microorganism preparations, various kinds of ingredients are added to stabilize the bacterial viability during production, distribution and consumption of food and feed, namely ingredients which are effective and have a viable stage in vivo in human or animal bodies. As for Bifidobacterium preparations, there are known: a preparation with added asparagine or sodium glutamate or soluble starch for storage at a high temperature (JP-C-43-3889); a gastric acid stable Bifidobacterium viable powder obtained by adding skim milk, starch hydrolysate, sodium glutamate, sodium ascorbate and a magnesium salt to a cultured medium of Bifidobacterium and lyophylizing (JP-A-60-188060); a process for preparing a powder preparation containing active Bifidobacterium which can be stored for a long term at ambient temperature which comprises mixing wetted or suspended Bifidobacterium with raw starch and lyophilizing (JP-A-63-12594); and a process for preparing a powder preparation containing Bifidobacterium which can be stored for a long term at ambient temperature and which can be kept with few deaths of viable microorganisms during drying, granulation and storage, which comprises suspending the wetted or suspended cells of Bifidobacterium in a dispersion medium containing soluble cyclodextrin and drying to powderize (JP-A-63-251080)

There is also known, though not as a food or feed, a process for the production of a viable preparation of stable and active viable microorganism which comprises adsorbing viable mycelia of Fusarium to a zeolite type base and drying, or suspending the viable mycelia in a dispersion medium containing as a main ingredient D-sorbitol with a small amount of a glutamate salt and lyophilizing (JP-A-63-227507).

Spore-forming viable microorganisms such as those of the genus Bacillus are relatively stable in dry and acid conditions by sporulation, and are used as a viable microorganisms feed (JP-A-53-6211). Among these Toyocerin (trade name, Bacillus cereus var. toyoi, generic abbr. Bacillus toyoi), Laclis (trade name, Bacillus coagulans), Glogen (trade name, Bacillus subtilis var. natto), Paciflor (trade name, Bacillus cereus IP-5832) and Bioplus 2B (trade name, Bacillus licheniformis, Bacillus subtilis) are known.

A viable microorganism preparation is commonly applied as a mixed feed in order to increase the growth and improve the feed efficiency of livestock by maintaining a balance of intestinal microbial flora, which play a nutritional action in the metabolism of nutrients and synthesis of vitamins. A viable microorganism preparation can only be effective when it reaches the digestive tract, and hence it is essential that the feed is stable in acid, alkaline and heating conditions. Spore-forming viable microorganisms belonging to the genus Bacillus are preferably used.

Pellet type feed has number of advantages; for example each pellet is a complete feed with homogenized feed components; the availability of nutrients in the feed and available energy is increased; the palatability of the feed is improved; body weight gain and feed efficiency are improved; less bulk facilitates easy handling; feeding time and labour can be saved; and waste of feed by blowing wind and spilling is minimized. In view of these advantages a pellet type feed is quite popular. Since the pellets are manufactured by formulating and pelleting feed components, which have previously been powderized once, with pressure and steam by means of a pellet-mill, heat stable spore-forming microorganisms such as those of the genus Bacillus are partially destroyed and lose viability due to the effects of steam-heat, generating heat, pressure and water content. Hence there has been the disadvantage that the efficacy of the viable microorganisms is lost.

In order to manufacture a pellet type feed of a stabilized spore-forming viable microorganism preparation of specific microorganisms of the genus Bacillus, we have made number of experiments on pelleting in combination with prior known stabilizing agents for Bifidobacterium and other viable microorganisms and spore-forming viable microorganism preparations, and measured the viable counts, to try to obtain a stable spore-forming viable microorganism preparation. However sufficient results could not be obtained due to the death of microorganisms due to heating and pressure during the pelleting process. We have found quite surprisingly that by spray-drying or kneading-blow drying a particular spore-forming viable microorganism preparation belonging to the genus Bacillus and a carbohydrate component originating from a cereal, the decrease in viable count during the pelleting process or mixing with feed for livestock is significantly improved and stabilized.

The present invention provides a stabilized spore-forming viable microorganism preparation comprising a carbohydrate component obtainable from a cereal and a spore-forming viable microorganism which is Bacillus toyoi.

The present invention also provides a process for the production of a stabilized spore-forming microorganism preparation as defined above which comprises drying a carbohydrate component obtainable from a cereal and a spore-forming viable microorganism which is Bacillus toyoi in the presence of non-toxic aqueous medium.

The present invention further provides pellet suitable for use as an animal feed comprising a preparation as described above.

Bacillus toyoi has the trade name Toyocerin.

Examples of a carbohydrate component obtainable from a cereal are components from maize (Indian corn or corn) powder per se, for example crushed or powdered seeds of maize [Zea mays Linne (Gramineae)], optionally being dried, and commercially available cornflour (powdered corn) and corngrits (Indian meal, hominy, corn meal).

Other examples of carbohydrates from cereals are components from rice, for example de-fatted rice bran per se, namely a de-fatted, dried and if necessary crushed mixture of the seed coat, albumen and starch layer of threshed seeds of the rice plant [Oryza sativa Linne (Gramineae)] obtained during rice polishing.

Examples of starch are maize starch (cornstarch), rice starch, and wheat starch. These are a mixture of amylose and amylopectin, and hence any starch obtained from cereals can be used.

An example of wheat flour is a crushed and optionally dried seeds of Triticumsativum Lamarck - (Gramineae), and wheat middling or wheat bran.

An example of a carbohydrate from soy bean is, for example crushed and optionally dried seeds of Glycine max Merrill (Leguminosae), and de-fatted soybean flour, soybean meal, soybean mill-run or soybean cake.

These bases can be used, for example, by mixing one or more kinds of the base with the spore-forming viable microorganism to prepare an aqueous suspension, then spray-drying the suspension after pre-heating, for example to above 60 - 75°C, to a gelatinization temperature of the starch component from maize, or without pre-heating treatment, or by kneading the mixture and then drying with blowing. The dried mixture is pulverized to a powder using a crushing mill such as a speed-mill and granulated by an oscillating granulator to prepare a dried viable microorganism powder preparation A carbohydrate component obtainable from cereals such as rice bran, starch, wheat flour or soybean flour can also be used by mixing the suspension thereof directly, or a pre-heated gelatinized component thereof, with the spore-forming viable microorganism, then spray-drying or kneading and blow-drying.

The above exemplified base used in the present invention shows a stabilizing effect on microorganisms in a spore-forming viable microorganism preparation during pellet production, though the effect is slightly different depending on the kind of base and the drying conditions, so that spray-drying or blow-drying is performed with an aqueous suspension or heat-gelatinization. However any drying process provides the stabilizing effect which might be the result of heat-gelatinization of starch.

The viable microorganism formula feed obtained by mixing the dried powder viable microorganism preparation with an animal feed or fish feed is pelleted by a conventional method using a pelleting machine to obtain a pellet type feed. The admixed feed and ingredients can be selected according to the nature of the livestock, poultry or fish to prepare a proper formula feed and additives.

The mixing amount of the base, e.g. components from maize or de-fatted rice bran, starch, wheat powder or soybean flour, and spore-forming viable microorganisms can, for example, be at least 0.01 parts by weight of the base per one part by weight of microbial cells, usually 0.01 - 100 parts by weight, preferably 0.1 - 10 parts by weight. Viable counts below approximately $10^{13}$ - $10^{15}$ cells are contained in 1 kg of microbial cells.

Pelleting of viable microorganism formula feed can be by a conventional pelleting method. A mixture of dried powder of the viable microorganism preparation and formula feed or ingredients is pelleted using a pellet-mill (e.g. Type J10, Jooda Iron Work Co.). Pellet feed can be produced using dies for pelleting with supplying steam and thereafter drying. The hardness and size of the pellet should be selected according to the kind of animal feed. For example, in Japan, the preferred pellet hardnesses are: soft type for chickens (i.e. Monsant hardness approx. 2 - 5 kg), medium type for swine (i.e. Monsant hardness approx. 5 - 7 kg) and hard type for rabbits (i.e. Monsant hardness approx. 2 - 12 kg). These feeds for livestock, poultry, and other animals and fish are formed into pellets.

Under the pelleting process, a considerable amount of the viable microorganism admixed in the feeds is disrupted and heat-denatured to death due to hardening of the pellets by steam and high pressure. In the

4

case of pelleting of conventional spray-dried spore-forming viable microorganisms belonging to the genus <u>Bacillus</u>, the viable count is reduced to approx. 30% at high hardness, though viability depends on the hardness of pellets prepared. On the contrary a viable microorganism preparation of the present invention prepared by spray-drying shows a viable count amount of over 80%.

Accordingly, even at a pellet hardness of 8 - 10 kg, a viable count of at least 70%, preferably at least 80%, of the remaining viable microorganisms is said to be an advantageously improved stability.

T spore-forming viable microorganism powdered preparation of the present inventions, containing <u>Bacillus toyoi</u> and a carbohydrate component obtainable from a cereal such as maize flour, de-fatted rice bran, starch, wheat flour and soybean flour, can have the viable count of microorganisms therein maintained after pelleting, as compared with before pelleting, with an admixed for livestock or fish or a veterinary drug. Hence it is easy to apply the composition for feed economically. The preparation of the present invention can be applied, for example, in an amount of $0.2 \sim 2 \times 10^6$ cells/kg feed weight for animal feed, $0.2 \sim 1 \times 10^7$ cells/kg feed weight for fish feed, and $5 \times 10^6 \sim 2 \times 10^8$ cells/kg feed weight for veterinary drugs.

The following Examples further illustrate the present invention.

Example 1

A viable cell spore suspension of <u>Bacillus toyoi</u> (Toyocerin) (cell count: $2.1 \times 10^{10}$ cell/ml; microbial cells 10%) was suspended with cornflour (Sunny Maize Co.) as illustrated in Table 1, and spray-dried by means of a spray-dryer (KC-50, Ohkawara Kakoki Co.) to obtain a powdered viable microorganism preparation.

Spray-drying: intake temperature: 150°C, outlet temp: 90 °C atomizer: 10,000 rpm.

For control group-1, Toyocerin is mixed with calcium carbonate powder (Korocalso-WB, trade name: Shiraishi Calcium Co.) and spray-dried to obtain the product "Control-1".

Table 1

| Sample No. | cell suspension (ℓ) ( ) : cell weight (kg) | Base (kg) | Ratio of base : cell weight | Viable count in dried powder (cells/g) |
|---|---|---|---|---|
| 1-a | 20 (2) | 2 | 1 : 1 | $9.11 \times 10^{10}$ |
| 1-b | 20 (2) | 1 | 1 : 0.5 | $13.9 \times 10^{10}$ |
| 1-c | 20 (2) | 0.2 | 1 : 0.1 | $17.7 \times 10^{10}$ |
| 1-d | 20 (2) | 0.02 | 1 : 0.01 | $19.5 \times 10^{10}$ |
| Control-1 | 20 (2) | 10 | 1 : 5 | $3.0 \times 10^{10}$ |

Example 2

The samples obtained in Example 1 were mixed uniformly with feed at a viable count of approx. 1.1 × $10^6$ cells per gram to prepare a mash. Pellets were prepared using a pellet-mill (Jooda Iron Work Co. Type J10), dies diameter; 3.5 mm, with supplying steam, then with drying to obtain the pellet product. The stability of the viable microorganisms was checked by counting the viable counts in pellets as compared with those in the mash. Since the hardness of pellet depends on the variety of feed, hardness was selected as follows:. Hardness; high: (A) feed for rabbits (NRT-1S, Nisseiken Co.), Hardness; middle: (B) feed for swine (synthetic milk for late term, Nihon Haigo Shiryo Co.) and Hardness; slightly low: (C) feed for chickens (feed for chick, Nihon Haigo Shiryo Co.).

The hardness of pellets was measured using a Monsant hardness meter. The results are shown in Table 2, in which sample 1-d; viable counts, slightly improved, and samples 1-a, 1-b and 1-c; viable counts, significantly improved, as compared with a decrease in viable count in the control after pelleting. Specifically, a high hardness pellet shows a significantly improved viable count.

Accordingly, the stability of viable microorganisms in a pelleting process with feed was shown to be more improved in the powdered preparation obtained by spray-drying the mixture of cornflour in an amount of greater than 0.01 parts, preferably greater than 0.1 parts, per one part by weight of microbial cells.

Table 2

| Sample No. | Feed | Pellet hardness (kg) | Viable count in mash (cells/g) | Viable count in pellet (cells/g) | Viable ratio (%) |
|---|---|---|---|---|---|
| 1-a | A | 8.9 | $1.29 \times 10^8$ | $1.13 \times 10^8$ | 88 |
|     | B | 7.4 | $1.33 \times 10^8$ | $1.13 \times 10^8$ | 85 |
|     | C | 3.3 | $1.32 \times 10^8$ | $1.27 \times 10^8$ | 96 |
| 1-b | A | 9.6 | $1.01 \times 10^8$ | $0.807 \times 10^8$ | 80 |
|     | B | 5.8 | $1.17 \times 10^8$ | $0.958 \times 10^8$ | 82 |
|     | C | 3.9 | $1.06 \times 10^8$ | $1.06 \times 10^8$ | 100 |
| 1-c | A | 9.6 | $1.28 \times 10^8$ | $1.10 \times 10^8$ | 86 |
|     | B | 5.7 | $1.19 \times 10^8$ | $1.17 \times 10^8$ | 98 |
|     | C | 4.2 | $1.28 \times 10^8$ | $1.14 \times 10^8$ | 89 |
| 1-d | A | 9.2 | $1.13 \times 10^8$ | $0.659 \times 10^8$ | 58 |
|     | B | 7.2 | $1.06 \times 10^8$ | $0.704 \times 10^8$ | 66 |
|     | C | 4.0 | $1.10 \times 10^8$ | $0.968 \times 10^8$ | 88 |
| Control-1 | A | 9.1 | $1.05 \times 10^8$ | $0.359 \times 10^8$ | 34 |
|           | B | 6.2 | $1.01 \times 10^8$ | $0.548 \times 10^8$ | 54 |
|           | C | 3.9 | $1.32 \times 10^8$ | $1.03 \times 10^8$ | 78 |

Example 3

Cornflour (each 2 g and 20 g, respectively) suspended in water (200 ml) was heated at 85 °C to gelatinization. The gelatinized liquid was then cooled and kneaded well with admixed cells of Bacillus toyoi - (20 g, $2.1 \times 10^{10}$ cells/g), then spread on a plate and dried by blowing at room temperature or at 80°C. The dried product was crushed by a speed-mill and subsequently granulated by an oscillating granulator (32 mesh, oscillater) to prepare a viable microorganism preparation.

Further cornflour (20 g) suspended in water (200 ml) was mixed with the microbial cells (20 g) and kneaded to prepare the preparation. The results are shown in Table 3.

Table 3

| Sample No. | Cell weight (g) | Base (cornflour) weight (g) | Base (cornflour) condition of liquid | Ration of base : cell weight | Blow-drying | Viable count in dried powder (cells/g) |
|---|---|---|---|---|---|---|
| 3-a | 20 | 20 | paste | 1 : 1 | room temp. | $7.2 \times 10^{10}$ |
| 3-b | 20 | 2 | paste | 1 : 0.1 | room temp. | $13.5 \times 10^{10}$ |
| 3-c | 20 | 20 | paste | 1 : 1 | 80°C | $6.4 \times 10^{10}$ |
| 3-d | 20 | 20 | suspension | 1 : 1 | room temp. | $7.4 \times 10^{10}$ |

Example 4

Samples obtained in Example 3 were pelleted by the same process as Example 2. The viable count was measured and compared with that of the mash to check the stability of the viable microorganisms.

The results are shown in Table 4. The stability of samples 3-a, 3-b and 3-c, which are prepared by adding a pasty liquid of a preheated gelatinized cornflour, was observed to be improved as compared with control samples in the pelleting process. Contrary to that, sample 3-d, which was prepared only by suspending cornflour in water and drying, was observed to be unstable. Accordingly, in case of cornflour, pasty gelatinized liquid thereof subjected to heat-treatment was preferable for drying with microbial cells in the pelleting process, for good stability of viable microorganisms cells.

8

Table 4

| Sample No. | Feed | pellet hardness(kg) | Viable of count in mash(cells/g) | Viable count in pellet(cells/g) | Viable ration(%) |
|---|---|---|---|---|---|
| 3-a | A | 8.4 | $1.22 \times 10^8$ | $1.10 \times 10^8$ | 90 |
|  | B | 7.0 | $1.18 \times 10^8$ | $1.20 \times 10^8$ | 102 |
|  | C | 4.2 | $1.37 \times 10^8$ | $1.32 \times 10^8$ | 96 |
| 3-b | A | 11.2 | $1.30 \times 10^8$ | $1.12 \times 10^8$ | 86 |
|  | B | 6.8 | $1.26 \times 10^8$ | $1.20 \times 10^8$ | 95 |
|  | C | 4.8 | $1.21 \times 10^8$ | $1.24 \times 10^8$ | 102 |
| 3-c | A | 9.4 | $1.28 \times 10^8$ | $1.01 \times 10^8$ | 79 |
|  | B | 5.8 | $1.14 \times 10^8$ | $0.988 \times 10^8$ | 87 |
|  | C | 3.9 | $1.03 \times 10^8$ | $1.08 \times 10^8$ | 105 |
| 3-d | A | 10.9 | $1.17 \times 10^8$ | $0.510 \times 10^8$ | 44 |
|  | B | 7.4 | $1.12 \times 10^8$ | $0.704 \times 10^8$ | 63 |
|  | C | 4.1 | $1.04 \times 10^8$ | $0.973 \times 10^8$ | 94 |
| Control-1 | A | 9.1 | $1.05 \times 10^8$ | $0.359 \times 10^8$ | 34 |
|  | B | 6.2 | $1.01 \times 10^8$ | $0.548 \times 10^8$ | 54 |
|  | C | 3.9 | $1.32 \times 10^8$ | $1.03 \times 10^8$ | 78 |

Example 5

De-fatted rice bran (Nihon Seimaiseiyu Co.) was suspended or kneaded with a cell-suspension of Bacillus toyoi ($2.1 \times 10^{10}$ cells/ ml; microbial cells 10%) in the compositions shown in Table 5, and spray-dried according to the process of Example 1 or blow-dried according to that of Example 3 to obtain powdered viable microorganism preparations.

Table 5

| Sample No. | Cell suspension (ℓ) ( ) :cell weight (kg) | Base (kg) | Ratio of cells : base | Drying method | Viable count in dried powder (cells/g) |
|---|---|---|---|---|---|
| 5 — a | 0.2 (0.02) | 0.02 | 1 : 1 | room temp. blow-drying | $7.5 \times 10^{10}$ |
| 5 — b | 0.2 (0.02) | 0.02 | 1 : 1 | 80℃· blow-drying | $7.2 \times 10^{10}$ |
| 5 — c | 10 (1) | 1 | 1 : 1 | spray-drying | $9.6 \times 10^{10}$ |

Example 6

Pellets of the samples of Example 5 were produced by the same process as Example 2. The viable count was measured and compared with that of the mash for to check the stability of viable microorganisms.

The results are shown in Table 6. The stability of a powdered viable microorganism preparation using de-fatted rice bran produced by blow-drying and spray-drying in the pelleting process was superior. Accordingly, as for cornflour, rice bran was observed to provide stability for viable microorganisms in the pelleting process.

Table 6

| Sample No. | Feed | Pellet hardness (kg) | Viable count in mash (cells/g) | Viable count in pellet (cells/g) | Viable ratio (%) |
|---|---|---|---|---|---|
| 5-a | A | 9.6 | $1.23 \times 10^6$ | $1.11 \times 10^6$ | 90 |
|  | B | 7.3 | $1.16 \times 10^6$ | $1.05 \times 10^6$ | 91 |
|  | C | 4.8 | $1.30 \times 10^6$ | $1.31 \times 10^6$ | 101 |
| 5-b | A | 8.1 | $1.18 \times 10^6$ | $0.944 \times 10^6$ | 80 |
| 5-c | A | 8.2 | $1.18 \times 10^6$ | $1.02 \times 10^6$ | 86 |
|  | B | 4.8 | $1.09 \times 10^6$ | $1.04 \times 10^6$ | 95 |

Example 7

Starch (cornstarch, Wako Pure Chem. Co.) was suspended with a spore-cell suspension of Bacillus toyoi (Toyocerin, $2.7 \times 10^{10}$ cells/ml; microbial cells 10%) in the compositions illustrated in Table 7, and spray-dried using a spray-dryer (Ohkawara Kakoki Co., Type KC-50) to obtain a powdered viable microorganisms preparation.

Spray-drying:    intake temperature: 150°C, outlet temp: 90 °C atomizer: 10,000 rpm.

For control group-1, Toyocerin is mixed with calcium carbonate powder (Korocalso-WB, trade name: Shiraishi Calcium Co.) and spray-dried to obtain the product "Control-2".

11

Table 7

| Sample No. | Cell suspension (ℓ) ( ) :cell weight(kg) | Base (kg) | Ratio of cell : base | Viable count in dried powder (cells/g) |
|---|---|---|---|---|
| 7-a | 10 (1) | 1 | 1 : 1 | $12.6 \times 10^{10}$ |
| 7-b | 10 (1) | 0.5 | 1 : 0.5 | $14.9 \times 10^{10}$ |
| 7-c | 10 (1) | 0.1 | 1 : 0.1 | $17.0 \times 10^{10}$ |
| 7-d | 10 (1) | 0.01 | 1 : 0.01 | $17.4 \times 10^{10}$ |
| Control-2 | 10 (1) | 5 | 1 : 5 | $3.0 \times 10^{10}$ |

Example 8

The samples obtained in Example 7 were mixed uniformly with feed at a viable count of approx. $1.1 \times 10^6$ cells per gram to prepare a mash. Pellets were prepared using a pellet-mill (Jodda Iron Work Co. Type J10), dies diameter; 3.5 mm, with supplying steam, then drying to obtain the pellet product. The stability of the viable microorganisms was checked by counting the viable counts in pellets as compared with those in the mash. Since the hardness of pellet depends on the variety of feed, the hardness was selected as follows: Hardness; high: (A) feed for rabbits (NRT-1S, Nisseiken Co.), Hardness; middle: (B) feed for swine (synthetic milk for late term, Nihon Haigo Shiryo Co.) and Hardness; slightly low: (C) feed for chickens (feed for chick, Nihon HaigoShiryo Co.).

The hardness of pellets was measured using a Monsant hardness meter. The results are shown in Table 8, in which the decrease in viable counts in all samples was significantly improved as compared with the decrease in viable count in the control group after pelleting. Specifically, a high hardness pellet shows significant improved viable counts in samples 7-a, 7-b and 7-c.

12

Accordingly, the stability of viable microorganisms in a pelleting process with feed was shown to more improved in the powdered preparation obtained by spray-drying a mixture of starch in an amount of greater than 0.01 parts, preferably greater than 0.1 parts, per one part by weight of microbial cells.

Table 8

| Sample No. | Feed | pellet hardness (kg) | Viable count in mash (cells/g) | Viable count in (cells/g) | Viable ratio(%) |
|---|---|---|---|---|---|
| 7-a | A | 9.5 | $1.14 \times 10^4$ | $0.941 \times 10^4$ | 83 |
| | B | 8.3 | $1.07 \times 10^4$ | $0.907 \times 10^4$ | 85 |
| | C | 5.4 | $1.17 \times 10^4$ | $0.992 \times 10^4$ | 85 |
| 7-b | A | 10.9 | $1.02 \times 10^4$ | $0.836 \times 10^4$ | 82 |
| | B | 6.4 | $1.02 \times 10^4$ | $0.888 \times 10^4$ | 87 |
| | C | 5.1 | $0.978 \times 10^4$ | $0.886 \times 10^4$ | 91 |
| 7-c | A | 10.2 | $1.08 \times 10^4$ | $0.882 \times 10^4$ | 82 |
| | B | 8.1 | $1.07 \times 10^4$ | $0.903 \times 10^4$ | 84 |
| | C | 5.7 | $1.03 \times 10^4$ | $1.02 \times 10^4$ | 99 |
| 7-d | A | 11.0 | $1.23 \times 10^4$ | $0.861 \times 10^4$ | 70 |
| | B | 7.4 | $1.22 \times 10^4$ | $1.02 \times 10^4$ | 84 |
| | C | 6.4 | $1.10 \times 10^4$ | $1.00 \times 10^4$ | 91 |
| Control-2 | A | 9.7 | $1.17 \times 10^4$ | $0.515 \times 10^4$ | 44 |
| | B | 7.0 | $1.01 \times 10^4$ | $0.566 \times 10^4$ | 56 |
| | C | 5.4 | $1.38 \times 10^4$ | $1.09 \times 10^4$ | 79 |

Example 9

Wheat flour (protein content: 9.3 - 9.5%, Daiya, trade name, Nihon Seifun Co.) was suspended with a spore-cell suspension of Bacillus toyoi (Toyocerin) at the composition illustrated in Table 9 and spray-dried to obtain a powdered viable microorganism preparation. In this Example the process was the same as that of Example 7.

13

Table 9

| Sample No. | cell suspension (ℓ) ( ) :cell weight(kg) | Base (kg) | Ratio of cell: base | Viable count in dried powder (cells/g) |
|---|---|---|---|---|
| 9 – a | 10 (1) | 1 | 1 : 1 | $13.2 \times 10^{10}$ |
| 9 – b | 10 (1) | 0.5 | 1 : 0.5 | $15.5 \times 10^{10}$ |
| 9 – c | 10 (1) | 0.1 | 1 : 0.1 | $16.5 \times 10^{10}$ |
| 9 – d | 10 (1) | 0.01 | 1 : 0.01 | $17.2 \times 10^{10}$ |

Example 10

Samples obtained in Example 9 were pelleted by the same process of Example 8. The viable count was measured and compared with that of the mash to check the stability of viable microorganisms, to obtain the same result as that of starch in Example 8. The results are shown in Table 10, in which preferable results are obtained.

## Table 10

| Sample No. | Feed | pellet hardness (kg) | Viable count in mash (cells/g) | Viable count in pellet(cells/g) | Viable ratio(%) |
|---|---|---|---|---|---|
| 9-a | A | 8.6 | $0.946 \times 10^8$ | $0.870 \times 10^8$ | 92 |
|  | B | 6.0 | $0.950 \times 10^8$ | $0.856 \times 10^8$ | 90 |
|  | C | 5.3 | $1.03 \times 10^8$ | $0.952 \times 10^8$ | 92 |
| 9-b | A | 9.2 | $1.03 \times 10^8$ | $0.921 \times 10^8$ | 89 |
|  | B | 6.7 | $1.04 \times 10^8$ | $0.953 \times 10^8$ | 92 |
|  | C | 5.7 | $1.13 \times 10^8$ | $1.09 \times 10^8$ | 96 |
| 9-c | A | 7.5 | $1.25 \times 10^8$ | $1.09 \times 10^8$ | 87 |
|  | B | 6.2 | $1.35 \times 10^8$ | $1.27 \times 10^8$ | 94 |
|  | C | 5.5 | $1.22 \times 10^8$ | $1.14 \times 10^8$ | 93 |
| 9-d | A | 8.7 | $1.39 \times 10^8$ | $1.01 \times 10^8$ | 73 |
|  | B | 5.8 | $1.16 \times 10^8$ | $0.961 \times 10^8$ | 83 |
|  | C | 5.4 | $1.13 \times 10^8$ | $1.02 \times 10^8$ | 90 |
| Control-2 | A | 9.7 | $1.17 \times 10^8$ | $0.515 \times 10^8$ | 44 |
|  | B | 7.0 | $1.01 \times 10^8$ | $0.566 \times 10^8$ | 56 |
|  | C | 5.4 | $1.38 \times 10^8$ | $1.09 \times 10^8$ | 79 |

Example 11

Soybean flour (Sunrichflour Showa, trade name, Showa Sangyo Co.), which was produced by a process wherein soybean seeds were defatted, dried and crushed to pass through a 80 mesh sieve, was suspended with a spore-cell suspension of Bacillus toyoi (Toyocerin) at the composition illustrated in Table 11 and spray-dried to obtain a powdered viable microorganism preparation. In this Example the process was the same as that of Example 7. Further lactose (sample 11-e, Pharmatose, trade name, De Melk-industrie Veghel bv, importer: Iwaki Co.) was suspended in water and spray-dried in the same way.

15

Table 11

| Sample No. | cell suspension (ℓ) ( ) :cell weight(kg) | Base (kg) | Ratio of cells: base | Viable count in dried powder(cells/g) |
|---|---|---|---|---|
| 11-a | 10 (1) | 1 | 1 : 1 | $11.6 \times 10^{10}$ |
| 11-b | 10 (1) | 0.5 | 1 : 0.5 | $14.3 \times 10^{10}$ |
| 11-c | 10 (1) | 0.1 | 1 : 0.1 | $16.0 \times 10^{10}$ |
| 11-d | 10 (1) | 0.01 | 1 : 0.01 | $17.5 \times 10^{10}$ |
| 11-e | 10 (1) | 0.5 | 1 : 0.5 | $14.0 \times 10^{10}$ |

Example 12

Samples obtained in Example 11 were pelleted by the same process of Example 8. The viable count was measured and compared with that of the mash to check the stability of viable microorganisms to obtain the same result as for starch in Example 8. No advanced effect was observed in the lactose (sample 11-e in the table). The results are shown in Table 12, in which preferable results are obtained.

Table 12

| Sample No. | Feed | Pellet hardness (kg) | Viable count in mash (cells/g) | Viable count in pellet (cells/g) | Viable ratio(%) |
|---|---|---|---|---|---|
| 11-a | A | 8.8 | $1.16 \times 10^4$ | $1.14 \times 10^4$ | 98 |
| | B | 5.7 | $1.05 \times 10^4$ | $0.996 \times 10^4$ | 95 |
| | C | 5.2 | $1.07 \times 10^4$ | $1.04 \times 10^4$ | 97 |
| 11-b | A | 9.4 | $1.04 \times 10^4$ | $0.915 \times 10^4$ | 88 |
| | B | 6.4 | $0.940 \times 10^4$ | $0.885 \times 10^4$ | 94 |
| | C | 4.5 | $1.23 \times 10^4$ | $1.22 \times 10^4$ | 99 |
| 11-c | A | 8.7 | $0.951 \times 10^4$ | $0.857 \times 10^4$ | 90 |
| | B | 6.1 | $1.06 \times 10^4$ | $0.902 \times 10^4$ | 85 |
| | C | 5.2 | $1.11 \times 10^4$ | $0.933 \times 10^4$ | 84 |
| 11-d | A | 8.2 | $1.16 \times 10^4$ | $0.823 \times 10^4$ | 71 |
| | B | 6.6 | $1.03 \times 10^4$ | $0.840 \times 10^4$ | 82 |
| | C | 5.0 | $1.11 \times 10^4$ | $0.922 \times 10^4$ | 83 |
| Control-2 | A | 9.7 | $1.17 \times 10^4$ | $0.515 \times 10^4$ | 44 |
| | B | 7.0 | $1.01 \times 10^4$ | $0.566 \times 10^4$ | 56 |
| | C | 5.4 | $1.38 \times 10^4$ | $1.09 \times 10^4$ | 79 |
| 11-e | B | 5.7 | $1.35 \times 10^4$ | $0.830 \times 10^4$ | 61 |

Example 13

Starch (refer to Example 7), cornflour (refer to Example 9), soybean flour (refer to Example 11) and hydroxypropyl methylcellulose (TC-5, Shinetsu Chem. Co.), each 2 g, suspended in water (200 ml) were heated at 85 °C for gelatinization. The gelatinized liquid was cooled and kneaded well with admixed cells of Bacillus toyoi (20 g, 2.5 × 10¹⁰ cells/g), then spread on a plate and dried by blowing at room temperature. The dried product was crushed by a speed-mill and subsequently granulated by an oscillating granulator (32 mesh, oscillater) to prepare viable microorganism preparations.

The results are shown in Table 13. In addition, carboxymethyl ethylcellulose as well as hydroxypropyl methylcellulose shows no effect for stabilization.

Table 13

| Sample No. | cell weight (g) | Base | | Ratio of cells: base | Viable count in dried powder (cells/g) |
|---|---|---|---|---|---|
| | | substance | weight (g) | | |
| 13-a | 20 | starch | 2 | 1 : 0.1 | $17.5 \times 10^{10}$ |
| 13-b | 20 | wheat flour | 2 | 1 : 0.1 | $18.8 \times 10^{10}$ |
| 13-c | 20 | soybean flour | 2 | 1 : 0.1 | $17.0 \times 10^{10}$ |
| 13-d | 20 | hydroxymethylcellulose | 2 | 1 : 0.1 | $16.8 \times 10^{10}$ |

Example 14

Samples obtained in Example 13 were pelleted by the same process as that of Example 8 to produce pellets. The viable count was measured and compared with that of the mash to check the stability of viable microorganisms.

The results are shown in Table 14. The stability of samples 13-a, 13-b and 13-c, which were samples using a pasty liquid of a pre-heated gelatinized base, was observed to be improved as compared with control samples in the pelleting process. On the contrary, the stability of sample 13-d was not so improved.

Table 14

| Sample No. | Feed | Pellet hardness (kg) | Viable count in mash (cells/g) | Viable count in Pellet (cells/g) | Viable ratio (%) |
|---|---|---|---|---|---|
| 13-a | A | 8.8 | $1.28 \times 10^6$ | $1.11 \times 10^6$ | 87 |
|  | B | 7.4 | $1.12 \times 10^6$ | $1.14 \times 10^6$ | 102 |
|  | C | 4.7 | $0.998 \times 10^6$ | $0.889 \times 10^6$ | 89 |
| 13-b | A | 9.2 | $1.31 \times 10^6$ | $1.21 \times 10^6$ | 92 |
|  | B | 6.8 | $1.09 \times 10^6$ | $0.937 \times 10^6$ | 86 |
|  | C | 5.7 | $1.03 \times 10^6$ | $0.989 \times 10^6$ | 96 |
| 13-c | A | 10.0 | $0.983 \times 10^6$ | $0.806 \times 10^6$ | 82 |
|  | B | 6.5 | $1.26 \times 10^6$ | $1.17 \times 10^6$ | 93 |
|  | C | 5.2 | $1.31 \times 10^6$ | $1.27 \times 10^6$ | 97 |
| Control-2 | A | 9.7 | $1.17 \times 10^6$ | $0.515 \times 10^6$ | 44 |
|  | B | 7.0 | $1.01 \times 10^6$ | $0.566 \times 10^6$ | 56 |
|  | C | 5.4 | $1.38 \times 10^6$ | $1.09 \times 10^6$ | 79 |
| 13-d | A | 8.4 | $1.15 \times 10^6$ | $0.624 \times 10^6$ | 54 |

## Claims

1. A stabilised spore-forming viable microorganism preparation comprising a carbohydrate component obtainable from a cereal and a spore-forming viable microorganism which is Bacillus toyoi.

2. A preparation according to claim 1 wherein the carbohydrate component is obtainable from a cereal which is rice, wheat, barley, rye, oat, soy bean or maize.

3. A preparation according to claim 3 wherein the carbohydrate component is obtainable from maize (Zea mays).

4. A preparation according to any one of the preceding claims wherein the carbohydrate component is a starch or other polysaccharide.

5. A preparation according to any one of the preceding claims which comprises more than 0.01 parts by weight of the carbohydrate component per part by weight of the spore-forming viable microorganism.

6. A process for the production of a stabilized spore-forming microorganism preparation as claimed in any one of the preceding claims which comprises drying a carbohydrate component obtainable from a cereal and a spore-forming viable microorganism which is Bacillus toyoi in the presence of non-toxic aqueous medium.

19

**7.** A process according to claim 6 which comprises heat treating the carbohydrate component one or more times.

**8.** A process according to claim 7 which comprises preheat treating the carbohydrate component.

**9.** A process according to claim 7 or 8 wherein the heat-treatment is heat-drying.

**10.** Pellet suitable for use as an animal feed comprising a preparation as claimed in any one of claims 1 to 5.

**Patentansprüche**

**1.** Stabilisierte, sporenbildende, lebensfähige Mikroorganismen-Zubereitung mit einer Kohlenhydrat-Komponente, erhältlich von Getreide und einem sporenbildenden, lebensfähigen Mikroorganismus, bei dem es sich um *Bacillus toyoi* handelt.

**2.** Zubereitung nach Anspruch 1, wobei die Kohlenhydrat-Komponente von Getreide erhältlich ist, bei dem es sich um Reis, Weizen, Gerste, Roggen, Hafer, Sojabohnen oder Mais handelt.

**3.** Zubereitung nach Anspruch 3, wobei die Kohlenhydrat-Komponente von Mais (*Zea mays*) erhältlich ist.

**4.** Zubereitung nach einem der vorhergehenden Ansprüche, wobei die Kohlenhydrat-Komponente Stärke oder ein anderes Polysaccharid ist.

**5.** Zubereitung nach einem der vorhergehenden Ansprüche, wobei die Zubereitung mehr als 0,01 Gewichtsteile der Kohlenhydrat-Komponente pro Gewichtsteil des sporenbildenden, lebensfähigen Mikroorganismus umfaßt.

**6.** Verfahren zur Herstellung einer stabilisierten, sporenbildenden Mikroorganismus-Zubereitung gemäß einem der vorhergehenden Ansprüche, bei dem man eine Kohlenhydrat-Komponente, erhältlich von Getreide und einem sporenbildenden, lebensfähigen Mikroorganismus, bei dem es sich um *Bacillus toyoi* handelt, in Gegenwart eines nicht-toxischen wässerigen Mediums trocknet.

**7.** Verfahren nach Anspruch 6, bei dem man die Kohlenhydrat-Komponente einmal oder mehrmals wärmebehandelt.

**8.** Verfahren nach Anspruch 7, bei dem man die Kohlenhydrat-Komponente einer Wärmevorbehandlung unterwirft.

**9.** Verfahren nach Anspruch 7 oder 8, bei dem man eine Wärmetrocknung als Wärmebehandlung durchführt.

**10.** Pellets, geeignet zur Verwendung als Tierfutter mit einer Zubereitung gemäß einem der Ansprüche 1 bis 5.

**Revendications**

**1.** Préparation de microorganismes viables formant des spores statibilisés comprenant un composant carbohydrate pouvant être obtenu à partir d'une céréale et d'un microorganisme viable formant des spores qui est Bacillus toyoi.

**2.** Préparation selon la revendication 1 dans laquelle le composant carbohydrate peut être obtenu à partir d'une céréale qui est du riz, du blé, de l'orge, du seigle, de l'avoine, des graines de soja ou du maïs.

**3.** Préparation selon la revendication 2 dans laquelle le composé carbohydrate est obtenu à partir de maïs (Zea mays).

4. Préparation selon l'une quelconque des revendications précédentes dans laquelle le composant carbohydrate est de l'amidon ou un autre polysaccharide.

5. Préparation selon l'une quelconque des revendications précédentes qui comprend plus de 0,01 partie en poids du composant carbohydrate par partie en poids du microorganisme viable formant des spores.

6. Procédé pour la production d'une préparation de microorganismes formant des spores stabilisés telle que revendiquée dans l'une quelconque des revendications précédentes qui comprend le séchage d'un composant carbohydrate qui peut être obtenu à partir d'une céréale et d'un microorganisme viable formant des spores qui est Bacillus toyoi en présence d'un milieu aqueux non toxique.

7. Procédé selon la revendication 6 qui comprend le traitement thermique du composant carbohydrate une ou plusieurs fois.

8. Procédé selon la revendication 7 qui comprend le prétraitement thermique du composant carbohydrate.

9. Procédé selon la revendication 7 ou 8 dans lequel le traitement thermique est un séchage à la chaleur.

10. Comprimé approprié pour une utilisation en tant que nourriture pour animaux comprenant une préparation telle que revendiquée dans l'une quelconque des revendications 1 à 5.